# EUROPEAN PATENT APPLICATION

(11) **EP 2 818 132 A1**
(43) Date of publication of application: **31.12.2014**
(21) Application number: 13751147.3
(22) Date of filing: 19.02.2013
(51) Int. Cl.: A61C 1/08, A61C 19/04

(54) **DENTAL HANDPIECE VIDEO IMAGE ACQUISITION APPARATUS, DENTAL HANDPIECE IMAGE CAPTURE DEVICE, DENTAL HANDPIECE, AND DENTAL HANDPIECE SYSTEM**

(30) Priority: 20.02.2012 JP 2012033500
(71) Applicant: Motoyama, Osamu, Tokyo 112-0006 (JP); GC Corporation, Tokyo 113-0033 (JP)
(72) Inventor: MOTOYAMA, Osamu, Tokyo 112-0006 (JP)
(74) Representative: Beck Greener
(86) International application number: PCT/JP2013/053942
(87) International publication number: WO 2013/125508

(57) **Abstract**

Provided is an image acquisition device for dental handpiece capable of acquiring a clear image even though cooling water is supplied. An image acquisition device (11) detachable to a dental handpiece body (1), the device including a light inlet part having a light acquisition port capable of acquiring light, a holding member (14, 15) holding the light inlet part to the dental handpiece body, wherein the light acquisition port can be held to the dental handpiece body by means of the holding member in such a manner that it can be shifted to a side which is away from an instrument than a plane of an instrument-mounting surface of a head (2) of the dental handpiece body and in a region outside of an outer peripheral surface of the head.

## Description

### BACKGROUND OF THE INVENTION

### Field of the Invention

The present invention relates to an image acquisition device for dental handpiece, an imaging apparatus for dental handpiece, a dental handpiece and a dental handpiece system that are used for dental treatment.

### Description of the Related Art

In a dental treatment, an instrument is attached to a dental handpiece body, and the instrument cuts a tooth. At this time, it is preferred that a practitioner proceeds the cutting while observing the cutting state. In particular, it is important that a shape of a dental prosthesis configured to be attached to the tooth is matched in a good accuracy with the tooth after cutting, which is known to largely affect to a lifetime of the dental prosthesis. Therefore, it is very important that the treatment is carried out in a good accuracy while observing the cutting state in a clearer condition.

As a method for observing a state of a tooth in treatment, there is a method of directly seeing the tooth and a method of using a mirror. However, a dead angle is formed when the tooth is directly seen, and it is difficult to use a mirror together with the dental handpiece at the same time.

Patent Document 1 discloses a technique in which a portion to take an image is provided to a dental handpiece in a manner to be arranged to an instrument-mounting part and the image is taken from the portion by means of a camera via optical fibers and displayed. It is said that, according to this, since a tooth to be cut can be displayed to a display device, it is possible to carry out a treatment while observing a part to be cut.

### Citation List

### Patent Literatures

Patent Document 1: Japanese Patent Application Laid-Open No. H09-56730

### SUMMARY OF THE INVENTION

### Problems to be Solved by the Invention

In cutting a biological body such as a tooth, since a heat is generated by the cutting, cooling water is supplied in order to protect the biological body. Recently, in view of increasing a cooling efficiency, water mist is sprayed in many cases.

However, with the technique disclosed in Patent Document 1, it is difficult to obtain a clear image since the sprayed water adheres to a portion to take an image.

Considering the above problems, an object of the present invention is to provide an image acquisition device for dental handpiece capable of obtaining a clear image even though cooling water is supplied. Also, the present invention provides an imaging apparatus for a dental handpiece, a dental handpiece and a dental handpiece system that are capable of obtaining a clear image even though cooling water is supplied.

### Means for Solving the Problems

Hereinafter, the present invention will be described. In order to make the present invention easy to understand, reference numerals given in the accompanying drawings are shown here in parentheses. However, the present invention is not limited to this.

A first aspect of the present invention is an image acquisition device (11) detachable to a dental handpiece body (1), including a light inlet part (13a) provided with a light acquisition port (13d) capable of acquiring light and a holding member (14, 15) holding the light inlet part to the dental handpiece body, wherein, the light acquisition port is capable of being held to the dental handpiece body by means of the holding member in such a manner that it can be shifted to a side which is away from an instrument (3) than a plane of an instrument-mounting surface (2a) of a head (2) of the dental handpiece body and in a region outside of an outer peripheral surface of the head of the dental handpiece body.

A second aspect of the present invention is the image acquisition device (11) according to the first aspect, wherein the light acquisition port (13d) is capable of being held in a region within a radius of 25 mm of a rotation axis of the instrument in planer view.

Here, the planer view means a view in which a direction of the rotation axis of the instrument is defined as a back and front direction.

A third aspect of the present invention is an imaging apparatus (10) for dental handpiece including: the image acquisition device (11) according to the first or second aspect; a photoelectric conversion means (23) receiving incident light from the light inlet part (13a) and converting the light to an electrical signal; and an image processing means (25) receiving the electrical signal converted by the photoelectric conversion means and carrying out an image processing.

A fourth aspect of the present invention is a dental handpiece including a dental handpiece body (1) and an image acquisition device (11) to be provided to the dental handpiece body, wherein the image acquisition device includes a light inlet part (13a) provided with a light acquisition port (13d) capable of acquiring light, and the light acquisition port is capable of being held to the dental handpiece body by means of the holding member in such a manner that it can be shifted to a side which is away from an instrument (3) than a plane of an instrument-mounting surface (2a) of a head (2) of the dental handpiece body and in a region outside of an outer peripheral surface of the head of the dental handpiece body.

A fifth aspect of the present invention is the dental handpiece according to the fourth aspect, wherein the light acquisition port (13d) is held in a region within a radius of 25 mm of a rotation axis of the instrument (3) in planer view.

A sixth aspect of the present invention is a dental handpiece system including: the dental handpiece according to the fourth or fifth aspect; a photoelectric conversion means (23) receiving incident light from the light inlet part (13a) and converting the light to an electrical signal; and an image processing means (25) receiving the electrical signal converted by the photoelectric conversion means and carrying out an image processing.

### Effects of the Invention

According to the present invention, it is possible to obtain a clear image in cutting a tooth even though cooling water is supplied.

### BRIEF DESCRIPTION OF THE DRAWINGS

Fig. 1 is a view to explain one embodiment, the view schematically showing a structure of a dental handpiece system;
Fig. 2 is an enlarged view of a surrounding area of a head in Fig. 1;
Fig. 3A is a plane view of the surrounding area of the head to show a structure thereof;
Fig. 3B is a plane view of the surrounding area of the head to explain a region where a light acquisition port is to be disposed;
Fig. 4A is a view showing light acquisition and light emission means from an instrument side in Fig. 1;
Fig. 4B is a view showing a cross section taken along an IVb-IVb line shown in Fig. 4A in a same viewpoint as in Fig. 1;
Fig. 5 is a view to explain another embodiment, the view schematically showing a structure of a dental handpiece system;
Fig. 6A is a view showing light acquisition and light emission means from an instrument side in Fig. 5;
Fig. 6B is a view showing the light acquisition and light emission means in a same viewpoint as in Fig. 5.

### DETAILED DESCRIPTION OF THE INVENTION

The functions and benefits of the present invention will be apparent from the following description of modes for carrying out the invention. Hereinafter, the invention will be described based on embodiments shown in the drawings. However, the invention is not limited by these embodiments.

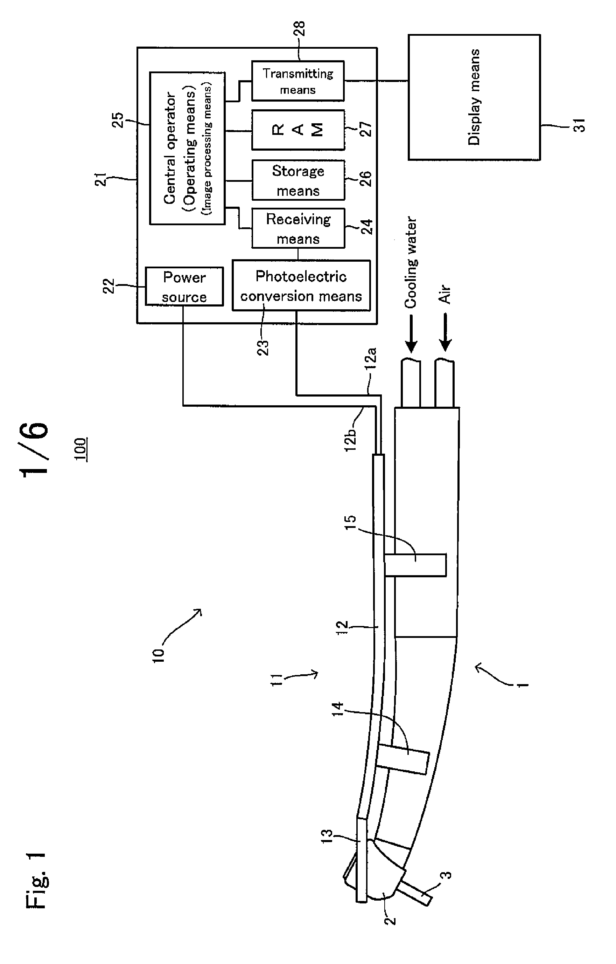

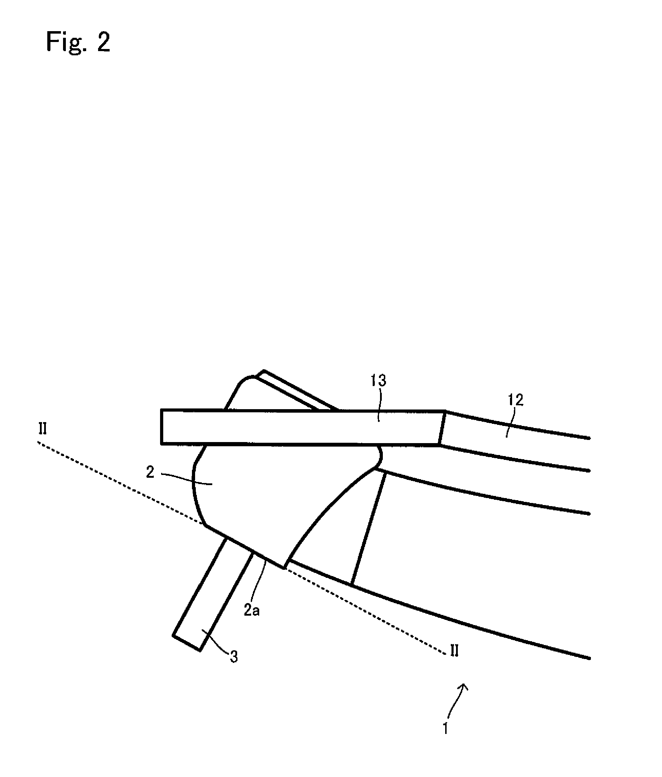

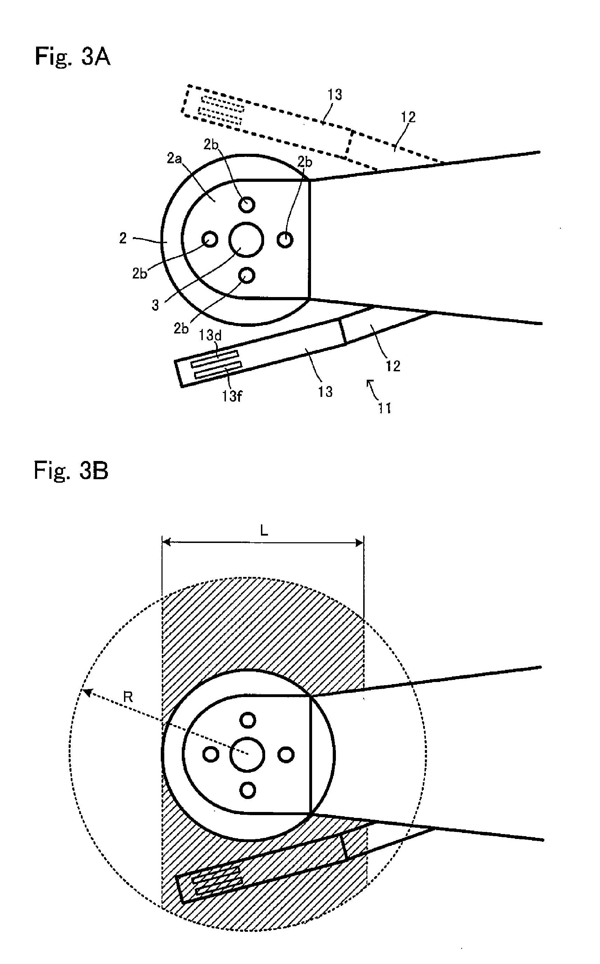

Fig. 1 is a view to explain one embodiment, the view schematically showing a structure of a dental handpiece system 100. Fig. 2 is an enlarged view of a surrounding area of a head 2 of a dental handpiece body 1. Fig. 3 includes enlarged views of the surrounding area of the head 2 of the dental handpiece body 1, the views being seen from a side where an instrument 3 is to be mounted in planer view. Fig. 3A shows a structure and Fig. 3B is a view to explain a region where a light acquisition port is to be disposed.

The dental handpiece system 100 is configured including the dental handpiece body 1 and an imaging apparatus 10 for dental handpiece.

A known dental handpiece body can be applied as the dental handpiece body 1 in this embodiment. That is, the dental handpiece body 1 includes a head 2, and the head 2 includes an instrument-mounting part on an instrument-mounting surface 2a of one surface of the head 2, to which the instrument 3 such as a dental drill is to be mounted. A turning force is applied to the instrument 3 by means of introducing air to a flow path provided inside the dental handpiece body 1.

Also, as shown in Fig. 3A, on the instrument-mounting surface 2a of the head 2, four cooling water ejection ports 2b are provided. The cooling water ejection ports 2b are connected to the flow path provided inside the dental handpiece body 1, and cooling water is ejected from the cooling water ejection ports 2b by means of introducing water to the flow path.

Here, the embodiment of the dental handpiece body 1 is shown as one example, however, the dental handpiece body 1 is not limited to this, and a known dental handpiece body can be applied as mentioned above.

The imaging apparatus 10 for dental handpiece includes an image acquisition device 11, a processing means 21 and a display means 31.

The image acquisition device 11 is configured including a light guiding means 12, a light acquisition and light emission means 13 and holding members 14 and 15.

The light guiding means 12 is a cable-like member configured such that light is guided inside the guiding means 12. At least two light guiding paths are formed inside the light guiding means 12. That is, a light guiding path 12a for image and a light guiding path 12b for light source light. Specific configuration of guiding light to form the light guiding path 12a for image and the light guiding path 12b for light source light is not particularly limited, but as an example, optical fibers can be used.

Here, the light guiding means 12 preferably has flexibility and can keep its posture at a predetermined position. This makes it possible to change the posture of the image acquisition device 11 to a desired posture and keep the desired posture.

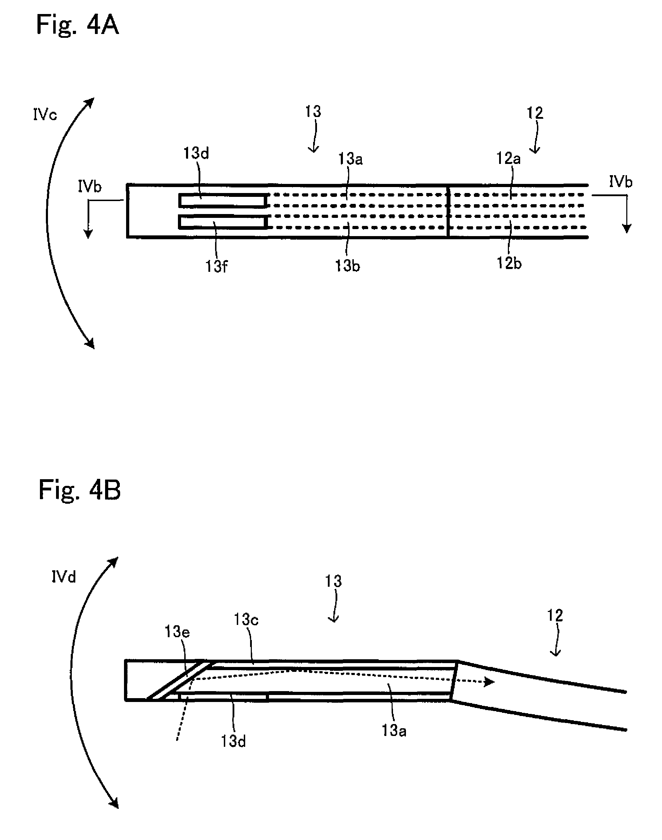

The light acquisition and light emission means 13 is disposed to a tip of one side of the light guiding means 12, as can be seen from Figs. 1 and 2. Fig. 4 includes views focusing a surrounding area of the light acquisition and light emission means 13 of the image acquisition device 11. Fig. 4A is a view of the light acquisition and light emission means 13 seen from a side of the instrument 3 (that is, a view seen in the same viewpoint as in Fig. 3A), and Fig. 4B is a cross-sectional view taken along IVb-IVb line shown in Fig. 4A in the same viewpoint as in Fig. 1.

As can be seen from Fig. 4A, the light acquisition and light emission means 13 includes the light inlet part 13a and the light source light outlet part 13b.

The light inlet part 13a has a function to let light from an oral cavity enter to obtain an image to be taken and guide the light to the light guiding path 12a for image of the light guiding means 12 described above. Therefore, the light inlet part 13a is configured to be communicated with the light guiding path 12a for image.

On the other hand, the light source light outlet part 13b has a function to receive light source light from the light guiding part 12b for light source light of the light guiding means 12 described above and to emit the light as an illumination light for oral cavity.

Therefore, the light source light outlet part 13b is configured to be communicated with the light guiding path 12b for light source light.

In order to form the light inlet part 13a and the light source light outlet part 13b described above, the light acquisition and light emission means 13 has the following configuration.

The light acquisition and light emission means 13 includes an outer peripheral wall part 13c in the light inlet part 13a, as can be seen from Fig. 4B. The outer peripheral wall part 13c is a tubular member, and the light inlet part 13a is formed inside the tubular shape where light from the inside of the oral cavity enters. Therefore, it is preferred that an inner surface of the outer peripheral wall part 13c has a high optical reflectivity. To achieve this configuration, the outer peripheral wall part 13c can be made of a metal and the inner surface can be configured to be a mirror surface for example.

A part of the outer peripheral wall portion 13c is cut off to form a light acquisition port 13d. The light acquisition port 13d is a member where light transmits so that the light from the inside of the oral cavity can be obtained to the inside of the tubular shape of the outer peripheral wall part 13c. Therefore, a glass or resin which has translucency can be disposed thereto in addition to simply making a hole.

Also, a reflecting member 13e is provided to the opposite side of the outer peripheral wall part 13c from a side where is connected to the light guiding means 12. The reflecting member 13e is made of a material having a high optical reflectivity, and includes an inclined surface as can be seen from Fig. 4B to reflect the incident light from the light acquisition port 13d so as to make the light closer to a light guiding direction. The reflecting member 13e is not particularly limited as long as it is made of a material having a high optical reflectivity, and it can be made of a metal to use its mirror surface or can be made of a resin to use its total reflection generated by reflective index difference with air.

The light source light outlet part 13b is configured in the same manner as in the light inlet part 13a, whose illustration and detailed explanation are omitted. That is, the light source light outlet part 13b is configured including an outer peripheral wall part, a light source light emission port 13f and a reflecting member that are configured in the same manner as in the outer peripheral wall part 13c, the light acquisition port 13d and the like.

As can be seen from Fig. 1, the holding members 14 and 15 are members provided so as to project from the light guiding means 12, which makes it possible to attach and hold the light guiding means 12 and the light acquisition and light emission means 13 to the dental handpiece body 1. A specific configuration of the holding members 14 and 15 is not particularly limited as long as the holding members 14 and 15 can hold the light guiding means 12 and the light acquisition and light emission means 13, and a configuration in which the holding members 14 and 15 sandwich the dental handpiece body 1, a configuration in which the holding member 14 and 15 stick to the dental handpiece body 1 and the like can be exemplified.

Here, the holding members can have any configurations as long as the holding members can hold the light acquisition and light emission means to the dental handpiece, thus a holding means can be provided to the light acquisition and light emission means.

The image acquisition device 11 described above is attached and held to the dental handpiece body 1 in a manner described below to be made as a dental handpiece. That is, as can be seen from Figs. 1 to 3, the holding members 14 and 15 are attached to the dental handpiece body 1. At this time, the light acquisition port 13d of the light acquisition and light emission means 13 is disposed so as to satisfy the following conditions.

Firstly, as shown in Figs. 3A and 3B, the light acquisition port 13d of the light acquisition and light emission means 13 is disposed so as to face a side of the instrument 3.

Secondly, as shown in Fig. 2, the light acquisition port 13d is disposed in such a manner that it can be shifted to a side which is away from the instrument 3 than a plane of the instrument-mounting surface 2a shown by II-II.

Thirdly, as shown in Fig. 3B, preferably the light acquisition port 13d is disposed in a region outside of an outer peripheral surface of the head 2 in planer view and within a radius of R=25 mm of a rotation axis of the instrument 3. Here, the planer view means a viewpoint in which the rotation axis of the instrument 3 is a back and front direction as in Fig. 3B. As shown by hatching in Fig. 3B, a region within the radius of R=25 mm and within a length of L=30 mm from a tip of the head 2 toward the opposite end of the head 2 is more preferable.

The image acquisition device 11 capable of disposing the light acquisition port 13d as described above greatly reduces the problem that cooling water disturbs image acquisition, even though in the process of cutting a tooth and the like by means of the instrument 3 with the cooling water being sprayed from the cooling water ejection port 2b, whereby it is possible to obtain an appropriate image.

By disposing the light acquisition port 13d within the radius of R=25 mm described above, the light acquisition port 13d is not too much separated from the tooth, and a clearer image can be obtained as described above. Further, by disposing the light acquisition port 13d within the length of L=30 mm described above, the light acquisition port 13d is disposed either one side of the left/right of the dental handpiece body 1 having its longitudinal direction as an axis but not disposed beyond the head 2 which is the tip of the axis, therefore the image acquisition device 11 can also be adequately used in cutting a back tooth.

Also, when the light guiding means 12 is made of a material having flexibility as described above, the light acquisition and light emission means 13 can be held with its posture kept in a position shifted as shown by arrows IVc and IVd in Fig. 4. Therefore, like the light acquisition and light emission means 13 shown in Fig. 3A by dashed lines for example, the light acquisition and light emission means 13 can be moved from a position shown by solid lines to another position.

Going back to Fig. 1, another configuration of the imaging apparatus 10 for dental handpiece will be described.

A processing means 21 is a means to supply a light source light to the image acquisition device 11 and to process the obtained light. The processing means 21 is configured including a light source 22, a photoelectric conversion means 23, a receiving means 24, a central operator 25, a storage means 26, RAM 27 and a transmitting means 28.

The light source 22 is a light source providing light to illuminate the inside of an oral cavity, therefore, preferably white light. Any kind of light source can be applied without particular limitations, and a light-emitting diode (LED) is preferable in view of duration of life and energy conservation.

As can be seen from Fig. 1, the light source 22 is connected to the light guiding path 12b for light source light of the light guiding means 12.

The photoelectric conversion means 23 is a means to convert entered light to an electrical signal. Any configuration can be applied without any limitations as a specific configuration of such a means, and CCD, CMOS and the like can be exemplified. Therefore, as can be seen from Fig. 1, the photoelectric conversion means 23 is connected to the light guiding path 12a for image of the light guiding means 12 and convert the light guided thereto to an electrical signal.

The receiving means 24 is a member having a function to take the electrical signal based on the light from the inside of the oral cavity, the signal being from the photoelectric conversion means 23. The receiving means 24 is to be connected to the photoelectric conversion means 23. A so-called input port, input connector and the like are also included in the photoelectric conversion means 23. Both wired and wireless connection can be applied as its connection form.

The central operator 25 is a so-called CPU, and functions as an operating means for various means such as the image processing means. That is, the central operator 25 performs every program stored in the storage means 26 which functions as a storage medium, and carries out an operation and outputs predetermined results.

When the central operator 25 functions as an image processing means, the central operator 25 takes the electrical signal from the receiving means 24 and carries out an operation to generate an image signal as a result of the operation. At this time, in addition to displaying the received signal as an image as it is, the central operator 25 can add an effect if needed, such as enlarging a part of the image, displaying a certain part in a manner to stand out the part, measuring sizes on the display and the like. This improves convenience for a practitioner. A known program can be applied to the program for such image processing.

Also, the central operator 25 is connected to other members such as the receiving means 24, storage means 26, RAM 27, transmitting means 28 and the like that are provided to the processing means 21, and configured to control them based on the program.

The storage means 26 is a member functioning as a storage medium where the every program and data that are the basis of the operation to be carried out by the central operator 25 are stored. In addition, the storage means 26 can function as a storing means to store images and patient data to be stored.

RAM 27 is a member functioning as a working area of the operation by the central operator 25 and a temporary storage means of data. RAM 27 can be configured by SRAM, DRAM, a flash memory and the like in the same manner as in a known RAM.

The transmission means 28 is a member having a function to transmit a needed signal from obtained results to the display means 31. A so-called output port, output connector and the like are included in the transmission means 28. Both wired and wireless forms can be applied for the transmission.

The display means 31 is a means connected to the transmission means 28 of the processing means 21 to display an image based on information obtained from the transmission means 28, and a various monitors and the like can be applied.

According to the image acquisition device 11 for dental handpiece, the imaging apparatus 10 for dental handpiece, the dental handpiece and the dental handpiece system 100 having configurations described above, it is possible to cut a tooth and the like as follows.

Regarding the dental handpiece body 1, the mounted instrument 3 rotates by means of introducing air, and at the same time cooling water flows in to be ejected from the cooling water ejection port 2b. This makes it possible to cut a tooth and the like while cooling.

In this embodiment, the light source 22 of the processing means 21 is additionally turned on. A light is accordingly emitted from the light source 22, and the light is guided to the image acquisition device 11 connected to the light source 22 and is emitted from the light source light emission port 13f thereby illuminating the inside of the oral cavity. Specifically, the light emitted from the light source 22 is guided in the light guiding path 12b for light source light to reach the light source light outlet part 13b provided to the light acquisition and light emission means 13, and reflected by the reflecting member to be emitted from the light source light emission port 13f.

By illuminating the inside of the oral cavity as described above, it becomes possible to obtain an image of the inside of the oral cavity. That is, as shown by an arrow of dashed line in Fig. 4B, the reflected light from the illuminated oral cavity enters the light inlet part 13a from the light acquisition port 13d of the light acquisition and light emission means 13, and is reflected by the reflecting member 13e to enter the light guiding path 12a for image of the light guiding means 12. Thereafter, the light from the inside of the oral cavity is guided in the light guiding path 12a for image to generate an image by means of the processing means 21, whereby displaying the image to the display means 31.

As described above, according to the imaging apparatus 10 for dental handpiece, by attaching the image acquisition device 11 to the dental handpiece body 1 to make a dental handpiece, it is possible to carry out a treatment while visually observing a cutting state of tooth and the like whose cutting is carried out with the instrument 3, by means of the display means 31. Here, since the light acquisition port 13d of the image acquisition device 11 of the imaging apparatus 10 for dental handpiece can be disposed to a predetermined position as described above, occurrence of poor visibility due to the effect from cooling water can be largely reduced, whereby it becomes possible to obtain a clear image of the cut portion even though the cooling water is used.

Also, if flexibility is given to the light guiding means 12, the position of the light acquisition port 13d can be adequately changed, which makes it possible to obtain a much clearer image and flexibly correspond to treatment content.

In addition to this, if an image easier to observe is displayed by enlarging the image by means of the processing means 21 and the like, details and a portion where could not be observed before since it was in a dead angle can be observed, whereby it is possible to improve accuracy of the treatment and easiness of work.

In this embodiment, an example in which one light acquisition and light emission means 13 is provided is explained, however, the present invention is not limited to this, and can be configured so as to have two or more light acquisition and light emission means each disposed to a different position to each other. According to this, although the numbers of the light guiding paths for image and the light guiding paths for light source need to be increased depending on the number of the light acquisition and light emission means, it becomes possible to visually observe the tooth from more different angles.

Also, in this embodiment, the present invention is explained with an example in which the image acquisition device 11 is detachable to the dental handpiece body 1. With this embodiment, the image acquisition device 11 can be removed from the dental handpiece body 1 when the dental hand piece is washed, whereby the washing of the dental handpiece body can be easily carried out as usual.

It should be noted that, the dental handpiece can be configured such that the image acquisition device 11 is integrated with the dental handpiece body 1. This makes it possible to make the dental handpiece more downsized.

Also, the light acquisition and light emission means 13 may be configured so as to be detachable to the light guiding means 12. This makes it possible to exchange the light acquisition and light emission means 13 thereby improving convenience.

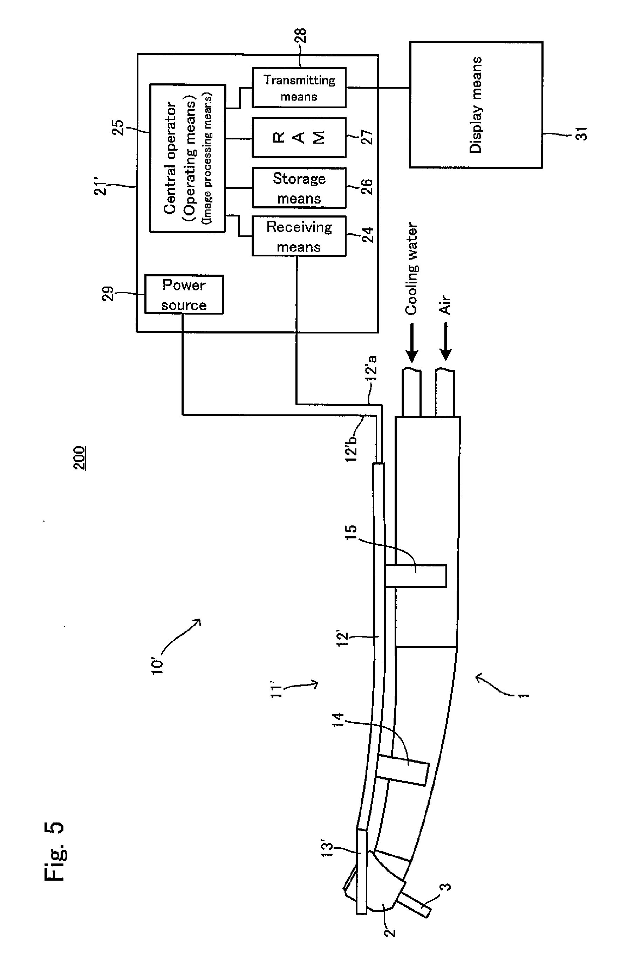

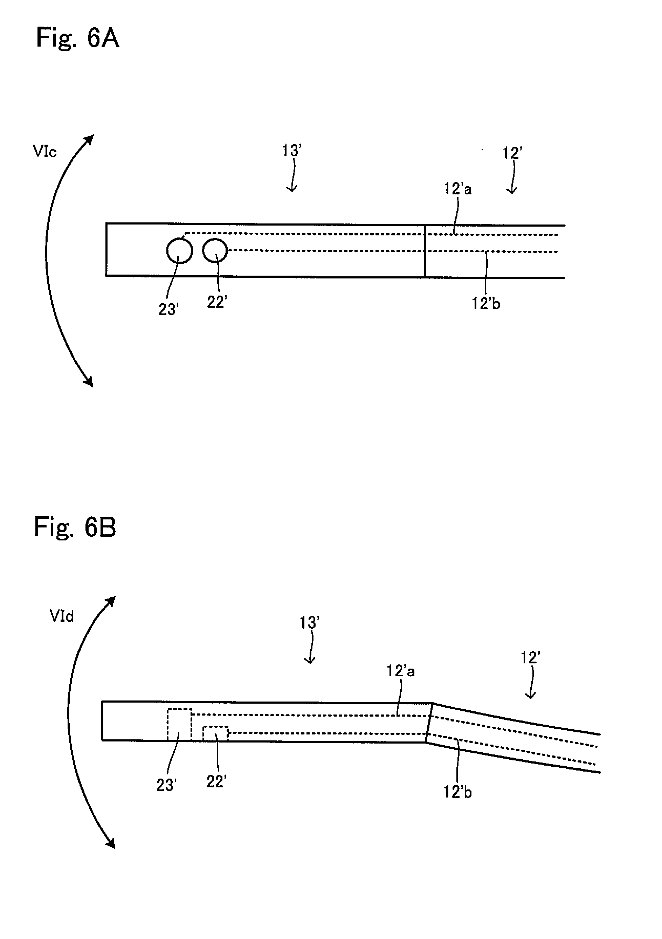

Figs. 5 and 6 include views to explain another embodiment, the views explaining a dental handpiece system 200. Fig. 5 corresponds to Fig. 1, and Fig. 6 corresponds to Fig. 4. Fig. 6A is a view showing a light acquisition and light emission means from a side of the instrument of Fig. 5, and Fig. 6B is a view showing the light acquisition and light emission means in the same viewpoint as in Fig. 5. Figs. 6A and 6B each shows an internal structure of the light acquisition and light emission means with dashed lines by seeing through it. The dental handpiece system 200 is configured including the dental handpiece body 1 and the imaging apparatus 10' for dental handpiece. Since the dental handpiece body 1 is same as that of the dental handpiece system 100 described above, the explanation will be omitted.

The imaging apparatus 10' for dental handpiece includes an image acquisition device 11', a processing means 21' and the display means 31.

The image acquisition device 11' is configured including a conduction means 12', a light acquisition and light emission means 13' and the holding members 14 and 15.

The conductive means 12' is a member configured including a conductive wire electrically connecting the light acquisition and light emission means 13' and the processing means 21' that are described below. The conduction means 12' includes a so-called conductive wire inside thereof, and at least two conductive wires are disposed. One of those is a conductive wire 12'a connecting a photoelectric conversion means 23' and the processing means 21' , and the other one is a conductive wire 12' b connecting a light source 22' and the processing means 21'.

Here, it is preferable that the conduction means 12' has flexibility to keep its posture at a predetermined position. This makes it possible to change a posture of the image acquisition device 11' to a desired posture and keep the desired posture.

As can be seen from Figs. 5, 6A and 6B, the light acquisition and light emission means 13' is disposed to a tip of one side of the conduction means 12'.

As can be seen from Figs. 6A and 6B, the light acquisition and light emission means 13' includes thereinside the photoelectric conversion means 23' including a light inlet part provided with a light acquisition port to emit light from inside of an oral cavity, and the light source 22' including a light source light outlet part.

The photoelectric conversion means 23' is a means letting in the light from the inside of the oral cavity which is an image to be taken and converting the light to an electrical signal. The converted signal is sent to the processing means 21' by means of the conductive wire 12'a. A specific configuration of the photoelectric conversion means 23' is not particularly limited, and a device including CCD, CMOS and the like can be exemplified.

On the other hand, the light source 22' is a means receiving a power source from the processing means 21' via the conductive wire 12'b and emitting an illumination light. Therefore, the light source 22' is preferably white light. A specific configuration of the light source 22' is not particularly limited, and LED can be exemplified in view of duration of life and energy conservation.

As described above, the photoelectric conversion means 23' and the light source 22' are directly disposed to the light acquisition and light emission means 13' in this embodiment.

Since the configurations of the holding members 14 and 15 and attachment and holding of the image acquisition device 11' to the dental handpiece body 1 by means of the holding members 14 and 15 are same as in the image acquisition device 11 describe above, the explanation will be omitted.

Here, when the conduction means 12' is made of a material having flexibility, it is possible to move the light acquisition and light emission means 13' and hold its posture as shown by arrows VIc and VId in Figs. 6A and 6B. Therefore, the light acquisition and light emission means 13' can be moved from one position to another position.

The processing means 21' is a means supplying a power source to the image acquisition device 11' and processing the electrical signal based on the light from the inside of the oral cavity. The processing means 21' is configured including a power source 29, the receiving means 24, the central operator 25, the storage means 26, RAM 27 and the transmitting means 28.

The power source 29 is a power source supplying electricity to the power source 22'. As can be seen from Fig. 5, the power source 29 is connected to the conductive wire 12'b of the conduction means 12'.

The receiving means 24 is a member having a function to take the electrical signal from the photoelectric conversion means 23' and connected to the photoelectric conversion means 23' via the conduction wire 12'a. A so-called input port, input connector and the like are also included in the receiving means 24. Both wired and wireless connections can be applied as its connection form.

Since the central operator 25, the storage means 26, RAM 27, the transmitting means 28 and the display means 31 are same as in the dental handpiece system 100 described above, their explanation will be omitted.

With the image acquisition device 11' for dental handpiece, the imaging apparatus 10' for dental handpiece, the dental handpiece and the dental handpiece system 200 each having the configuration as described above, the same effect as in the dental handpiece system 100 described above is exerted.

The dental handpiece system 100 and the dental handpiece system 200 are each described above, however, the present invention is not limited to these configurations. For example, the present invention can be configured such that the light source 22 is employed as a light source and the photoelectric conversion means 23' is employed as a photoelectric conversion means, or can be configured such that the light source 22' is employed as a light source and the photoelectric conversion means 23 is employed as a photoelectric conversion means.

### Description of the Reference Numerals

- 1: dental handpiece body
- 2: head
- 2a: instrument-mounting surface
- 2b: cooling water ejection port
- 3: instrument
- 10, 10': imaging apparatus for dental handpiece
- 11, 11': image acquisition device
- 12: light guiding means
- 12': conduction means
- 13, 13': light acquisition and light emission means
- 13a: light inlet part
- 13b: light source light outlet part
- 13c: outer peripheral wall part
- 13d: light acquisition port
- 13e: reflecting member
- 13f: light source light emission port
- 14, 15: holding member
- 21, 21': processing means
- 22, 22': light source
- 23, 23': photoelectric conversion means
- 24: receiving means
- 25: central operator (image processing means)
- 26: storage means
- 27: RAM
- 28: transmitting means
- 29: power source
- 31: display means
- 100, 200: dental handpiece system

## Claims

1. An image acquisition device detachable to a dental handpiece body, the image acquisition device comprising:
a light inlet part having a light acquisition port capable of acquiring light; and
a holding member holding the light inlet part to the dental handpiece body,
wherein:
the light acquisition port is capable of being held to the dental handpiece body by means of the holding member in such a manner that it can be shifted to a side which is away from an instrument than a plane of an instrument-mounting surface of a head of the dental handpiece body and in a region outside of an outer peripheral surface of the head of the dental handpiece body.

2. The image acquisition device according to claim 1, wherein the light acquisition port is capable of being held in a region within a radius of 25 mm of a rotation axis of the instrument in planer view.

3. An imaging apparatus for dental handpiece comprising:
the image acquisition device according to claim 1 or 2;
a photoelectric conversion means receiving incident light from the light inlet part and converting the light to an electrical signal; and
an image processing means receiving the electrical signal converted by the photoelectric conversion means and carrying out an image processing.

4. A dental handpiece comprising:
a dental hanpiece body; and
an image acquisition device to be provided to the dental handpiece body,
wherein:
the image acquisition device comprises a light inlet part having a light acquisition port capable of acquiring light; and
the light acquisition port is capable of being held to the dental handpiece body by means of the holding member in such a manner that it can be shifted to a side which is away from an instrument than a plane of an instrument-mounting surface of a head of the dental handpiece body and in a region outside of an outer peripheral surface of the head of the dental handpiece body.

5. The dental handpiece according to claim 4, wherein the light acquisition port is held in a region within a radius of 25 mm of a rotation axis of the instrument in planer view.

6. A dental handpiece system comprising:
the dental handpiece according to claim 4 or 5,
a photoelectric conversion means receiving incident light from the light inlet part and converting the light to an electrical signal; and
an image processing means receiving the electrical signal converted by the photoelectric conversion means and carrying out an image processing.
